# EUROPEAN PATENT SPECIFICATION

(11) **EP 1 268 837 B1**
(45) Date of publication and mention of the grant of the patent: **15.07.2009**
(21) Application number: 01919389.5
(22) Date of filing: 21.03.2001
(51) Int. Cl.: C12N 15/90, A01K 67/027, A01K 67/033, A01H 1/00

(54) **METHOD OF GENERATING TRANSGENIC MAMMALS USING TRANSPOSONS**
METHODE ZUR HERSTELLUNG VON TRANSGENE SÄUGETIERE MITTELS TRANSPOSONS
PROCEDE DE PRODUCTION de MAMMIFERES TRANSGENIQUES A L'AIDE DE TRANSPOSONS

(30) Priority: 21.03.2000 GB 0006753; 07.04.2000 US 195678 P
(43) Date of publication of application: 02.01.2003
(73) Proprietor: Minos Biosystems Limited, Smallwood, Sandbach, Cheshire CW11 2XB (GB); Erasmus University Medical Center Rotterdam, 3015 GD Rotterdam (NL)
(72) Inventor: SAVAKIS, Charalambos, 711 10 Heraklion (GR); GROSVELD Frank, 3065 GE Rotterdam (NL)
(74) Representative: Harding, Charles Thomas
(86) International application number: PCT/EP2001/003341
(87) International publication number: WO 2001/071019

(56) References cited:
- EP-A- 0 955 364
- WO-A-00/17343
- WO-A-02/13602
- WO-A-92/01370
- WO-A-99/09817
- US-A- 5 614 398
- HONMA MARY A ET AL: "High-frequency germinal transposition of Ds-ALS in Arabidopsis." PROCEEDINGS OF THE NATIONAL ACADEMY OF SCIENCES OF THE UNITED STATES, vol. 90, no. 13, 1993, pages 6242-6246, XP002172889 1993 ISSN: 0027-8424
- COOLEY L ET AL.: "Insertional mutagenesis of the Drosophila genome with single P elements" SCIENCE, vol. 239, 1988, pages 1121-1128, XP001008878
- LOUKERIS T G ET AL: "Introduction of the transposable element Minos into the germline of Drosophila melanogaster" PROCEEDINGS OF THE NATIONAL ACADEMY OF SCIENCES OF USA,NATIONAL ACADEMY OF SCIENCE. WASHINGTON,US, vol. 92, October 1995 (1995-10), pages 9485-9489, XP002163150 ISSN: 0027-8424
- HANDLER AM, HARRELL RA: "Germline transformation of Drosophila melanogaster with the piggyBac transposon vector" INSECT MOL. BIOL., vol. 8, no. 4, 1999, pages 449-457, XP002172890
- KLINAKIS AG ET AL.: "Genome-wide insertional mutagenesis in human cells by the Drosophila mobile element Minos" EMBO REPORTS, vol. 1, no. 5, 2000, pages 416-421, XP002172891
- KLINAKIS A G ET AL: "Mobility assays confirm the broad host-range activity of the Minos transposable element and validate new transformation tools" INSECT MOLECULAR BIOLOGY,BLACKWELL SCIENTIFIC, OXFORD,,GB, vol. 9, no. 3, June 2000 (2000-06), pages 269-275, XP002163113 ISSN: 0962-1075

## Description

The present invention relates to transgenic organisms, and methods for producing such organisms. In particular, the invention relates to transgenic organisms which comprise one or more insertions of a transposable element, or transposon. The transposon is preferably the Minos transposon.

Transposons are genetic elements which are capable of "jumping" or transposing from one position to another within the genome of a species. They are widely distributed amongst animals, including insects.

The availability of genetic methodologies for functional genomic analysis is crucial for the study of gene function and genome organization of complex eukaryotes. Of the three "classical" model animals, the fly, the worm and the mouse, efficient transposon based insertion methodologies have been developed for *D. melanogaster* and for *C*. *elegans*. The introduction of *P* element mediated transgenesis and insertional mutagenesis in *Drosophila* (Spradling & Rubin (1982) Science 218, 341-347) transformed *Drosophila* genetics and formed the paradigm for developing equivalent methodologies in other eukaryotes. However, thee P element has a very restricted host range, and therefore other elements have been employed in the past decade as vectors for .gene transfer and/or mutagenesis in a variety of complex eukaryotes, including nematodes, plants, fish and a bird.

Minos is a transposable element derived from *Drosophila* (Franz and Savakis, (1991) NAR 19:6646). It is described in US patent 5,840,865, which is incorporated herein by reference in its entirety. The use of *Minos* to transform insects is described in the foregoing US patent.

*Mariner* is a transposon originally isolated from *Drosophila,* but since discovered in several invertebrate and vertebrate species. The use of *mariner* to transform organisms is described in International patent application WO99/09817.

*Hermes* is derived from the common housefly. Its use in creating transgenic insects is described in US patent 5,614,398, incorporated herein by reference in its entirety.

*PiggyBac* is a transposon derived from the baculovirus host *Trichplusia ni.* Its use for germ-line transformation of Medfly has been described by Handler et al., (1998) PNAS (USA) 95:7520-5.

European Patent Application 0955364 (Savakis et al.*,* the disclosure of which is incorporated herein by reference) describes the use of Minos to transform cells, plants and animals. The generation of transgenic mice comprising one or more Minos insertions is described.

International Patent Application WO99/07871 describes the use of the Tc1 transposon from *C*. *elegans* for the transformation of *C*. *elegans* and a human cell line.

The use of *Drosophila* P-elements in *D. melanogaster* for enhancer trapping and gene tagging has been described; see Wilson et al., (1989) Genes dev. 3:1301; Spradling et al., (1999) Genetics 153:135.

EP 0955364 discloses transposable elements which hybridize to the DNA sequence of Minos-12

Cooley et al., (1988), Science 239: 1121-1128 describes an insertional mutagenesis method in *Drosophila* using single P elements.

In the techniques described in the prior art, the use of the cognate transposase for inducing transposon jumping is acknowledged to be necessary. Transgenic animals, where described, have the transposase provided in *cis* or *trans*, for example by cotransformation with transposase genes.

### Summary of the Invention

We have now developed an improved protocol for the generation of transgenic animals using transposable elements as a genetic manipulation tool. In the improved protocol, the transposase function is provided by crossing of transgenic organisms in order to produce organisms containing both transposon and transposase in the required cells or tissues. The invention allows tissue-specific, regulatable transposition events to be used for genetic manipulation of organisms.

According to a first aspect of the invention, there is provided a method for generating a transgenic non-human mammalian organism, comprising the steps of:
(a) providing a first transgenic organism, which organism comprises, within at least a portion of its tissues or cells, as a result of transgenesis, one or more copies of a transposon;
(b) providing a second organism, which organism comprises, in the genome of at least a portion of its tissues or cells, as a result of transgenesis, a transposase or one or more copies of a gene encoding a transposase wherein the tranposase is expressed under the control of control sequences which permit regulation of the expression of the transposase; and
(c) crossing the organisms so as to obtain transgenic progeny which comprise, in at least a portion of their tissues or cells, both the transposon and the transposase.

The invention comprises the crossing of two transgenic organisms, wherein one organism comprises, as a result of transgenesis, one or more copies of a transposon; and the other organism comprises, as a result of transgenesis, one or more copies of the cognate transposase.

It is highly preferred that the transposon is Minos. The organism is a non-human mammal.

A "cognate" transposase, as referred to herein, is any transposase which is effective to activate transposition of the transposon, including excision of the transposon from a first integration site and/or integration of the transposon at a second integration site. Preferably, the cognate transposase is the transposase which is naturally associated with the transposon in its *in vivo* situation in nature. However, the invention also encompasses modified transposases, which may have advantageously improved activities within the scope of the invention.

The transposon may be a natural transposon. Preferably, it is a type-2 transposon, such as Minos. Most advantageously, it is Minos. Alternative transposons include mariner, Hermes and piggyBac.

The invention moreover relates to the use of modified transposons, which incorporate one or more heterologous coding sequences and/or expression control sequences. Such coding sequences may include selectable and/or unselectable marker genes, which permit the identification of transposons in the genome and cloning of the loci into which the transposons have been integrated. Suitable markers include fluorescent and/or luminescent polypeptides, such as GFP and derivatives thereof, luciferase, β-galactosidase, or chloramphenicol acetyl transferase (CAT).

In an advantageous embodiment, the transposase may be expressed in the transgenic organisms in a regulatable manner. This means that the activation of the transposon can be determined according to any desired criteria. For example, the transposase may be placed under the control of tissue-specific sequences, such that it is only expressed at desired locations in the transgenic organism. Such sequences may, for example, comprise tissue-specific promoters, enhancers and/or locus control sequences.

Moreover, the transposase may be placed under the control of one or more sequences which confer developmentally-regulated expression. This will result in the transposons being activated at a given stage in the development of the transgenic animal or its progeny.

Using the techniques of the invention, gene modification events can be observed at a very high frequency, due to the efficiency of mobilisation and insertion of transposons. Moreover, the locus of the modification may be identified precisely by locating the transposon insertion. Sequencing of flanking regions allows identification of the locus in databases, potentially without the need to sequence the locus. Moreover, the use of transposons provides a reversible mutagenesis strategy, such that modifications can be reversed in a controlled manner.

The transposon may be inserted into a gene. Preferably, the transposon is inserted into a highly transcribed gene, resulting in the localisation of said transposon in open chromatin. This increases the accessibility of the transposon which may result in increased transposition frequencies.

Moreover, the transposon may itself comprise, between the transposon ends, a highly-transcribed gene. This will cause activation of the chromatin structure into which the transposon integrates, facilitating access of the transposase thereto.

The transposon may be inserted into the gene by recombination. Furthermore, the transposon may be inserted into the gene by recombination in cells such as ES cells.

According to a second aspect of the invention, there is provided a method for detecting and characterising a genetic mutation in a transgenic organism, comprising the steps of:
(a) generating a transgenic non-human mammalian organism by a procedure according to the first aspect of the invention;
(b) characterising the phenotype of the transgenic organism;
(c) detecting the position of one or more transposon insertion events in the genome of the organism; and
(d) correlating the position of the insertion events with the observed phenotype, the position of the insertion events being indicative of the location of one or more gene loci connected with the observed phenotype.

The generation of genetic mutations in transgenic organisms as a result of transposon insertion after crossing of transgenic organisms according to the invention gives rise to novel phenotypic variations in the organisms, which can be traced back to insertion events in the genome of the organism. Transposon excisions characteristically result in the insertion of a small number of nucleotides into the host genome, left behind by the transposon and the recombination events associated with its insertion and subsequent excision. Small insertions may have small phenotypic effects, for example resulting from the insertion of a few amino acids into the sequence of a polypeptide. Alternatively, the effects may be more pronounced, possibly including the complete inactivation of a gene.

Transposon insertions are more likely to have significant phenotypic consequences, on the grounds that the insertion is much larger.

If a transposon is inserted into an intron of a gene, resulting in inactivation of the gene, its excision leads, in the majority of cases, to restoration of gene activity. Thus, the invention provides a reversible mutagenesis procedure, in which a gene can be inactivated and subsequently restored.

Insertion events may be detected by screening for the presence of the transposon, by probing for the nucleic acid sequence of the transposon. Excisions may also be identified by the "signature" sequence left behind upon excision.

In a preferred embodiment, transposons may be used to upregulate the expression of genes. For example, a transposon may be modified to include an enhancer or other transcriptional activation element. Mobilisation and insertion of such a transposon in the vicinity of a gene upregulates expression of the gene or gene locus. This embodiment has particular advantage in the isolation of oncogenes, which may be identified in clonal tumours by localisation of the transposon.

According to a third aspect, there is provided a method for isolating a gene which is correlated with a phenotypic characteristic in a transgenic animal, comprising the steps of:
(a) generating a transgenic non-human mammalian organism by a procedure according to the first aspect of the invention;
(b) characterising the phenotype of the transgenic organism;
(c) detecting the position of one or more transposon insertion events in the genome of the organism; and
(d) cloning the genetic loci comprising the insertions.

The invention provides clear advantages in functional genomics, since gene disruption or activation by transposon jumping is easily traced due to tagging by the transposon.

According to a fourth aspect, there is provided a method for isolating an enhancer in a transgenic non-human mammalian animal, comprising the steps of:
(a) generating a transgenic organism by a procedure according to the first aspect of the invention, wherein the transposon comprises a reporter gene under the control of a minimal promoter such that it is expressed at a basal level;
(b) assessing the level of expression of the reporter gene in one or more tissues of the transgenic organism;
(c) identifying and cloning genetic loci in which the modulation of the reporter gene is increased or decreased compared to the basal expression level; and
(d) characterising the cloned genetic loci.

According to a fifth aspect, there is provided a method for isolating an exon of an endogenous gene in a transgenic animal, comprising the steps of:
(a) generating a transgenic organism by a procedure according to the first aspect of the invention, wherein the transposon comprises a reporter gene which lack translation initiation sequences but includes splice acceptor sequences;
(b) identifying tissues of the organism in which the reporter gene is expressed;
(c) cloning the genetic loci comprising the expressed reporter gene.

The invention may be used to provided *in vivo* enhancer trap and exon trap functions, by inserting transposons which comprise marker genes which are modulated in their expression levels by the proximity with enhancers or exons. Suitable constructs for such applications are described in EP 0955364 and known in the art. Since transposon activation may be effected in a tissue-specific or developmentally regulated manner, the invention permits the trapping of enhancers and/or exons which are subject to similar regulation in the transgenic organism.

According to a sixth aspect, there is provided a method for modulating the expression of a gene in a non-human mammalian animal, comprising the steps of:
(a) generating a library of transgenic organisms according to the first aspect of the invention;
(b) selecting from said library one or more transgenic organisms in which the expression of a gene of interest is modulated as a result of one or more transposon insertion events.

Moreover transgenic animals suitable for crossing in a method according to the invention, and a transgenic organism comprising one or more copies of a heterologous transposon, said transgenic organism being free of nucleic acid sequences encoding the cognate transposase enzyme, and a transgenic organism encoding a transposase enzyme, said transgenic organism being free of the cognate transposon, are described herein.

### Description of the Figures

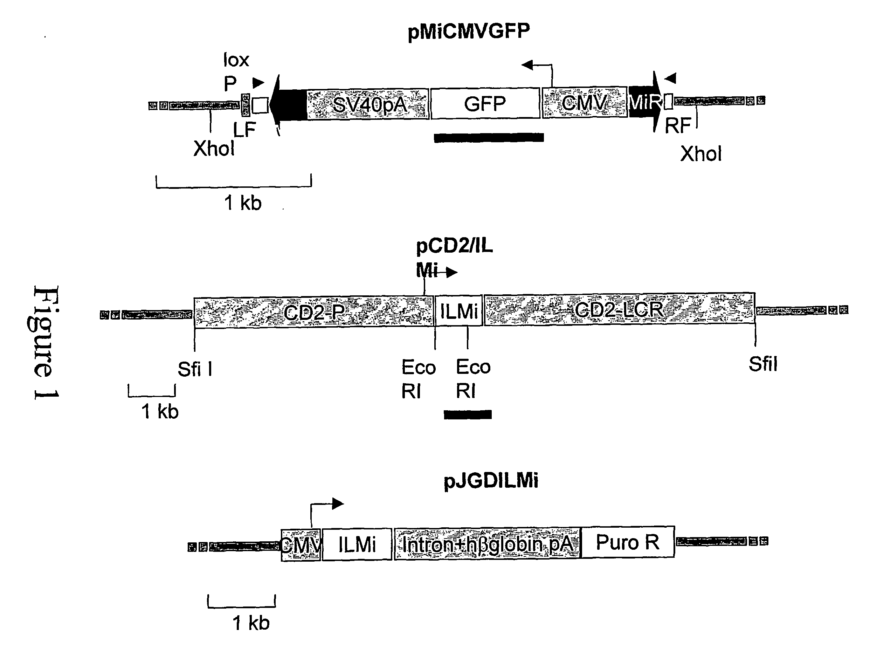

**Figure 1****.** *Minos* derived vectors. *Minos* inverted terminal repeats are shown as thick black arrows. White blocks outside these arrows indicate the sequences flanking the original *Minos* element in the *D. hydei* genome. Arrowheads indicate the positions of primers used to detect *Minos* excisions. Small arrows indicate the direction of transcription of the GFP and transposase genes. Black bars represent fragments used as probes.

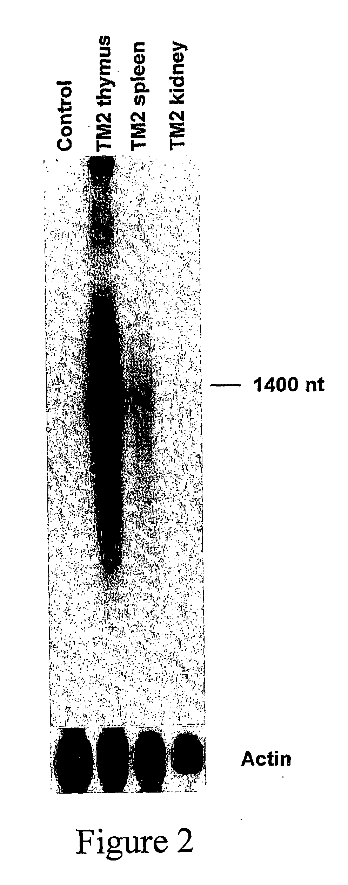

**Figure 2****.** Tissue specific expression of *Minos* transposase in transgenic mice. Northern blot analysis of thymus, spleen and kidney RNA isolated from TM2/+ mice (40-hr exposure). Control RNA is from thymus of a non-transgenic mouse. The lower panel shows the signal obtained upon re-hybridisation of the same filter with a mouse actin probe (3-hr exposure).

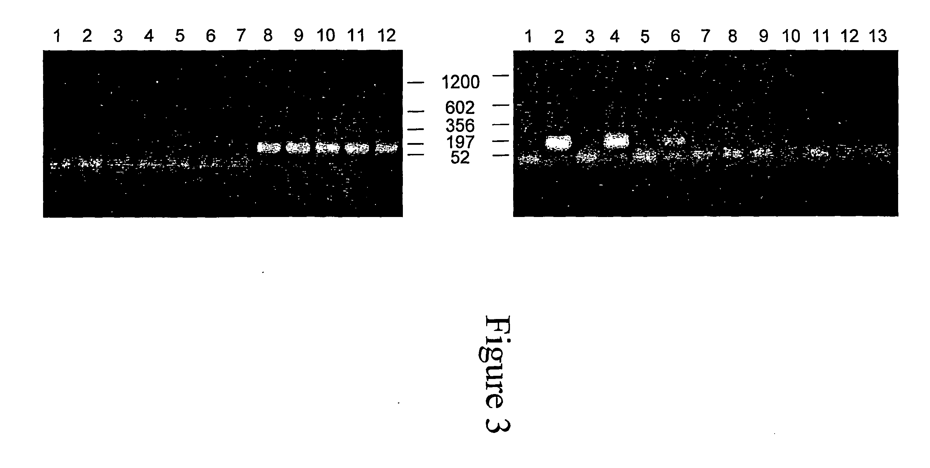

**Figure 3****.** Transposase dependent, tissue-specific excision of a *Minos* transposon in mice. Oligonucleotide primers flanking the transposon were used for PCR and the products were analysed by agarose gel electrophoresis. Left panel: Transposase-dependent excision in the thymus. Template DNA used: Lane 1, non transgenic; lane 2, TM2/+; lanes 3-7, MCG/+; lanes 8-12, MCG/+ TM2/+. Right panel: Excision in various tissues of transposase-expressing mice. Template DNA used: Lanes 1, 3, 5, 7, 9, 11, from MCG/+ mice. Lanes 2, 4, 6, 8, 10, 12, from MCG/+ TM2/+ mince. Lanes 1-2, thymus. Lanes 3-4, spleen. Lanes 5-6, liver. Lanes 7-8, kidney. Lanes 9-10, brain. Lanes 11-12, muscle. Lane 13, no DNA added.

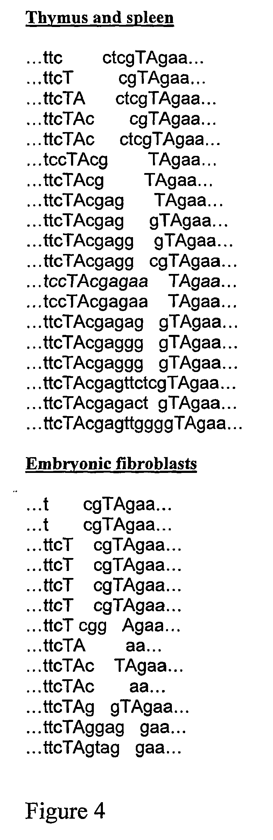

**Figure 4****.** Footprints left behind at chromosomal sites after *Minos* excision. DNA is extracted from thymus and spleen of a double transgenic mouse (top), or from an embryonic fibroblast cell line from a MCG/+ mouse after transfection with a transposase-expressing plasmid (bottom) and used as template for PCR with the flanking primers. PCR-amplified bands were cloned and 32 clones (19 from thymus and spleen and 13 from fibroblast cells) were sequenced. TA is the target site duplication. Nucleotides in red correspond to the ends of the transposon terminal repeats; nucleotides in blue are of unknown origin. The flanking nucleotides and TA repeats are aligned.

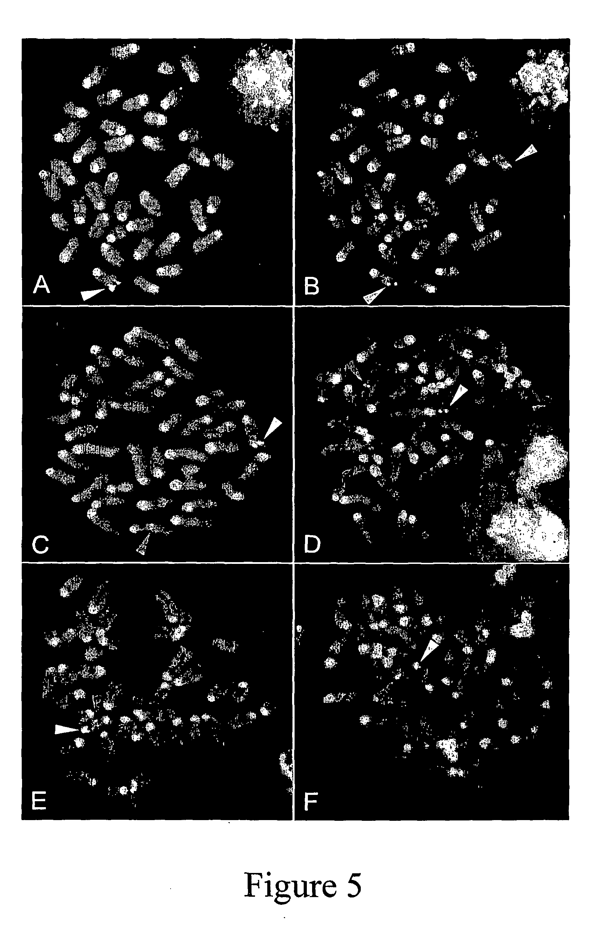

**Figure 5****.** FISH analysis of *Minos* transpositions in thymus and spleen. Chromosomes were stained with DAPI. Panels A and B are from the same MCG/+ metaphase nucleus, probed with a GFP and a telomere 14 specific probe, respectively. Panels C to F are nuclei probed with GFP. Panels C and D are from thymus and spleen respectively from the same MCG/+,TM2/+ mouse. Panels E and F are from spleen of two different MCG/+,TM2/+ mice. Yellow arrowheads indicate the original integration site of the transposon transgene, near the telomere of chromosome 14. Green arrowheads indicate the telomeres of chromosome 14. Red arrowheads indicate transposition events.

### Detailed Description of the Invention

Although in general the techniques mentioned herein are well known in the art, reference may be made in particular to Sambrook et al., Molecular Cloning, A Laboratory Manual (1989) and Ausubel et al., Short Protocols in Molecular Biology (1999) 4th Ed, John Wiley & Sons, Inc.

A transgenic organism of the invention is a non-human mammalian animal.

Particular examples of mammals include non-human primates, cats, dogs, ungulates such as cows, goats, pigs, sheep and horses and rodents such as mice, rats, gerbils and hamsters.

### Production of transgenic animals

Techniques for producing transgenic animals are well known in the art. A useful general textbook on this subject is Houdebine, Transgenic animals - Generation and Use (Harwood Academic, 1997) - an extensive review of the techniques used to generate transgenic animals from fish to mice and cows.

Advances in technologies for embryo micromanipulation now permit introduction of heterologous DNA into, for example, fertilised mammalian ova. For instance, totipotent or pluripotent stem cells can be transformed by microinjection, calcium phosphate mediated precipitation, liposome fusion, retroviral infection or other means, the transformed cells are then introduced into the embryo, and the embryo then develops into a transgenic animal. In a highly preferred method, developing embryos are infected with a retrovirus containing the desired DNA, and transgenic animals produced from the infected embryo. In a most preferred method, however, the appropriate DNAs are coinjected into the pronucleus or cytoplasm of embryos, preferably at the single cell stage, and the embryos allowed to develop into mature transgenic animals. Those techniques as well known. See reviews of standard laboratory procedures for microinjection of heterologous DNAs into mammalian fertilised ova, including Hogan et al., Manipulating the Mouse Embryo, (Cold Spring Harbor Press 1986); Krimpenfort et al., Bio/Technology 9:844 (1991); Palmiter et al., Cell, 41: 343 (1985); Kraemer et al., Genetic manipulation of the Mammalian Embryo, (Cold Spring Harbor Laboratory Press 1985); Hammer et al., Nature, 315: 680 (1985); Wagner et al., U.S. Pat. No. 5,175,385; Krimpenfort et al., U.S. Pat. No. 5,175,384, the respective contents of which are incorporated herein by reference

Another method used to produce a transgenic animal involves microinjecting a nucleic acid into pro-nuclear stage eggs by standard methods. Injected eggs are then cultured before transfer into the oviducts of pseudopregnant recipients.

Transgenic animals may also be produced by nuclear transfer technology as described in Schnieke, A.E. et al., 1997, Science, 278: 2130 and Cibelli, J.B. et al., 1998, Science, 280: 1256. Using this method, fibroblasts from donor animals are stably transfected with a plasmid incorporating the coding sequences for a polypeptide of interest under the control of regulatory sequences. Stable transfectants are then fused to enucleated oocytes, cultured and transferred into female recipients.

Analysis of animals which may contain transgenic sequences would typically be performed by either PCR or Southern blot analysis following standard methods.

By way of a specific example for the construction of transgenic mammals, such as cows, nucleotide constructs comprising a sequence encoding a DNA binding molecule are microinjected using, for example, the technique described in U.S. Pat. No. 4,873,191, into oocytes which are obtained from ovaries freshly removed from the mammal. The oocytes are aspirated from the follicles and allowed to settle before fertilisation with thawed frozen sperm capacitated with heparin and prefractionated by Percoll gradient to isolate the motile fraction.

The fertilised oocytes are centrifuged, for example, for eight minutes at 15,000 g to visualise the pronuclei for injection and then cultured from the zygote to morula or blastocyst stage in oviduct tissue-conditioned medium. This medium is prepared by using luminal tissues scraped from oviducts and diluted in culture medium. The zygotes must be placed in the culture medium within two hours following microinjection.

Oestrous is then synchronized in the intended recipient mammals, such as cattle, by administering coprostanol. Oestrous is produced within two days and the embryos are transferred to the recipients 5-7 days after oestrous. Successful transfer can be evaluated in the offspring by Southern blot.

Alternatively, the desired constructs can be introduced into embryonic stem cells (ES cells) and the cells cultured to ensure modification by the transgene. The modified cells are then injected into the blastula embryonic stage and the blastulas replaced into pseudopregnant hosts. The resulting offspring are chimeric with respect to the ES and host cells, and nonchimeric strains which exclusively comprise the ES progeny can be obtained using conventional cross-breeding. This technique is described, for example, in WO91/10741.

### Transposons

Minos transposons, and their cognate transposase, are described in detail in US patent 5,840,865 and European patent application EP 0955364, the disclosures of which are incorporated herein by reference. Minos transposons may be modified, for instance to insert one or more selectable marker genes for example as referred to herein, according to general techniques. Specific techniques for modifying Minos are set forth in EP 0955364.

### Marker genes

Preferred marker genes include genes which encode fluorescent polypeptides. For example, green fluorescent proteins ("GFPs") of cnidarians, which act as their energy-transfer acceptors in bioluminescence, can be used in the invention. A green fluorescent protein, as used herein, is a protein that fluoresces green light, and a blue fluorescent protein is a protein that fluoresces blue light. GFPs have been isolated from the Pacific Northwest jellyfish, *Aequorea victoria,* from the sea pansy, *Renilla reniformis*, and from *Phialidium gregarium*. (Ward et al., 1982, Photochem. Photobiol., 35: 803-808; Levine et al., 1982, Comp. Biochem. Physiol.,72B: 77-85). See also Matz, *et al*., 1999, ibid for fluorescent proteins isolated recently from Anthoza species (accession nos. AF168419, AF168420, AF168421, AF168422, AF168423 and AF168424).

A variety of *Aequorea*-related GFPs having useful excitation and emission spectra have been engineered by modifying the amino acid sequence of a naturally occurring GFP from Aequorea victoria (Prasher et al., 1992, Gene, 111: 229-233; Heim et al., 1994, Proc. Natl. Acad. Sci. U.S.A., 91: 12501-12504; PCT/US95/14692). As used herein, a fluorescent protein is an Aequorea-related fluorescent protein if any contiguous sequence of 150 amino acids of the fluorescent protein has at least 85% sequence identity with an amino acid sequence, either contiguous or non-contiguous, from the wild-type Aequorea green fluorescent protein (SwissProt Accession No. P42212). More preferably, a fluorescent protein is an Aequorea-related fluorescent protein if any contiguous sequence of 200 amino acids of the fluorescent protein has at least 95% sequence identity with an amino acid sequence, either contiguous or non-contiguous, from the wild type *Aequorea* green fluorescent protein of SwissProt Accession No. P42212. Similarly, the fluorescent protein may be related to *Renilla* or *Phialidium* wild-type fluorescent proteins using the same standards.

*Aequorea*-related fluorescent proteins include, for example, wild-type (native) *Aequorea victoria* GFP, whose nucleotide and deduced amino acid sequences are presented in Genbank Accession Nos. L29345, M62654, M62653 and others *Aequorea*-related engineered versions of Green Fluorescent Protein, of which some are listed above. Several of these, i.e., P4, P4-3, W7 and W2 fluoresce at a distinctly shorter wavelength than wild type.

### Identification of insertion and excision events

Minos transposons, and sites from which transposons have been excised, may be identified by sequence analysis. Minos typically integrates at a TA base pair, and on excision leaves behind a duplication of the target TA sequence, flanking the four terminal nucleotides of the transposon. The presence of this sequence, or related sequences, may be detected by techniques such as sequencing, PCR and/or hybridisation.

Inserted transposons may be identified by similar techniques, for example using PCR primers complementary to the terminal repeat sequences.

### Regulation of transposase expression

Coding sequences encoding the transposase may be operatively linked to regulatory sequences which modulate transposase expression as desired. Control sequences operably linked to sequences encoding the transposase include promoters/enhancers and other expression regulation signals. These control sequences may be selected to be compatible with the host organism in which the expression of the transposase is required. The term promoter is well-known in the art and encompasses nucleic acid regions ranging in size and complexity from minimal promoters to promoters including upstream elements and enhancers.

The promoter is typically selected from promoters which are functional in cell types homologous to the organism in question, or the genus, family, order, kingdom or other classification to which that organism belongs, although heterologous promoters may function - eg. some prokaryotic promoters are functional in eukaryotic cells. The promoter may be derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of α-actin, β-actin, tubulin) or, alternatively, a tissue-specific manner (such as promoters of the genes for pyruvate kinase). They may also be promoters that respond to specific stimuli, for example promoters that bind steroid hormone receptors. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

It is moreover advantageous for the promoters to be inducible so that the levels of expression of the transposase can be regulated. Inducible means that the levels of expression obtained using the promoter can be regulated. A widely used system of this kind in mammalian cells is the tetO promoter-operator, combined with the tetracycline/doxycycline-repressible transcriptional activator tTA, also called Tet-Off gene expression system (Gossen, M. & Bujard, H. (1992) Tight control of gene expression in mammalian cells by tetracycline responsive promoters. Proc. Natl. Acad. Sci. USA 89:5547-5551), or the doxycycline-inducible rtTA transcriptional activator, also called Tet-On system (Gossen, M., Freundlieb, S., Bender, G., Muller, G., Hillen, W. & Bujard, H. (1995) Transcriptional activation by tetracycline in mammalian cells. Science 268:1766-1769).

In the Tet-Off system, gene expression is turned on when tetracycline (Tc) or doxycycline (Dox; a Tc derivative) is removed from the culture medium. In contrast, expression is turned on in the Tet-On system by the addition of Dox. Procedures for establishing cell lines carrying the transcriptional activator gene and the Tet-regulatable gene stably integrated in its chromosomes have been described. For example see http://www.clontech.com/techinfo/manuals/PDF/PT3001-1.pdf. For example, the Tet-On system may be employed for tetracycline-inducible expression of Minos transposase in a transgenic animal. A doubly transgenic animal is generated by standard homologous recombination ES cell technology. Two constructs are used; first, a construct containing the rtTA gene under a constitutive promoter. An example of such construct is the pTet-On plasmid (Clontech) which contains the gene encoding the rtTA activator under control of the Cytomegalovirus immediate early (CMV) promoter. The rtTA transcriptional activator encoded by this construct is active only in the presence of Doxycycline. The second construct contains the Minos transposase gene under control of the tetracycline-response element, or TRE. The TRE consists of seven direct repeats of a 42-bp sequence containing the tet operator (tetO), and is located just upstream of the minimal CMV promoter, which lacks the enhancer elements normally associated . with the CMV immediate early promoter. Because these enhancer elements are missing, there is no "leaky" expression of transposase from the TRE in the absence of binding by rtTA. An example of such construct is the pTRE2 plasmid (Clontech) in the MCS of which is inserted the gene encoding Minos transposase. In cells stably transformed with the two constructs, rtTA is expressed by does not activate transcription of Minos transposase unless Doxycycline is administered to the animal.

Alternative inducible systems include or tamoxifen inducible transposase [a modified oestrogen receptor domain (Indra et al., Nucl Acid Res. 27, 4324-27, 1999) coupled to the transposase which retains it in the cytoplasm until tamoxifen is given to the culture], or a RU418 inducible transposase (operating under the same principle with the glucocorticoid receptor; see Tsujita et al., J. Neuroscience, 19, 10318-23, 1999).

In addition, any of these promoters may be modified by the addition of further regulatory sequences, for example enhancer sequences. Chimeric promoters may also be used comprising sequence elements from two or more different promoters described above.

The use of locus control regions (LCRs) is particularly preferred. LCRs are capable of conferring tightly-regulated tissue specific control on transgenes, and to greatly increase the fidelity of transgene expression. A number of LCRs are known in the art. These include the β-globin LCR (Grosveld et al., (1987) Cell 51:975-985); α-globin (Hatton et al., (1990) Blood 76:221-227; and CD2 (Festenstein et al., (1996) Science 271:1123-1125), plus immunoglobulins, muscle tissue, and the like.

Regulation of transposase and/or transposon expression may also be achieved through the use of ES cells. Using transformed ES cells to construct chimeric embryos, it is possible to produce transgenic organisms which contain the transposase genes or transposon element in only certain of their tissues. This can provide a further level of regulation.

The regulation of expression of transposase may induce excision of a transposon. This may be used to genetically manipulate an organism. As used herein, the term "genetically manipulate" refers to the manipulation of genes in an organism's genome and may include the insertion or excision of a gene or part of a gene.

The sequence of the transposase may be modified to optimise codon usage and thus, increase transposition frequencies. Optimisation of codon usage is a method well known in the art to increase the expression levels of a given gene.

The invention is further described, for the purpose of illustration, in the following examples.

### Examples

### Plasmid constructions

The helper plasmid CD2/ILMi is constructed by subcloning the transposase cDNA (Klinakis et al. (2000) EMBO reports 1, 416-421) as an *Xba*I-blunt fragment into the vector SVA(-). The SVA(-) vector is a derivative of the VA vector (Zhumabekov et al. (1995). J Immunol Methods 185, 133-140) with extended multiple cloning sites.

Transposon MiCMVGFP is constructed as follows: The plasmid pMILRTetR (Klinakis et al. (2000) Ins. Mol. Biol. 9, 269-275 (2000b) is cut with *BmnH* I and re-ligated to remove the tetracycline resistance gene between the *Minos* ends, resulting in plasmid pMILRΔ*Bam*H1. An *Asp*718/*Sac*I fragment from pMILRΔ*Bam* H1, containing the *Minos* inverted repeats and original flanking sequences from *D. hydei*, is cloned into plasmid pPolyIII-I-lox (created by insertion of the *loxP* oligo:
ATAACTTCGTATAGCATACATTATACGAAGTTAT
into the *Asp*718 site of the vector pPolyIII-I (accession No. M18131 ), resulting in plasmid ppolyMILRΔ*BamH*. The final construct (pMiCMVGFP, figure 1) used for the generation of transgenic mice, is created by inserting into the *Spe* I site of ppolyMILRΔ*Bam*H1 the 2.2 kb *Spe*I fragment from plasmid pBluescriptGFP, containing a humanised GFP gene (from Clontech plasmid pHGFP-S65T) driven by the CMV promoter and followed by the SV40 intervening sequence and polyadenylation signal.

Plasmid pJGD/TLMi (figure 1) is constructed as follows: A 1 kb *EcoR*V/*Not*I fragment containing the *Minos* transposase cDNA is cloned into *EcoR*V/*Not*I of plasmid pJG-3 (the puro variant of pJG-1; Drabek et al. (1997) Gene Ther. 4, 93-100 (1997). The resulting plasmid (pJGD/transposase) that carries a CMV promoter upstream of the transposase cDNA, an intron on with splice site and polyA from the human β globin gene and the puromycin resistance gene driven by PGK promoter and followed by the poly(A) signal from the bovine growth hormone gene is used as the transposase source in transfections of embryonic fibroblasts.

### Generation of transgenic mice.

The transposase-expressing TM2 mouse line is generated by injecting the 12.5 kb *Sfi*I fragment from the CD2/ILMi plasmid (figure 1) into CBA x C57 B1/10 fertilized oocytes. Transgenic founder animals are identified by Southern blotting of DNA from tail biopsies, using the 1 kb transposase cDNA fragment as a probe and crossed with F1 CBA x C57 B1/10 mice to generate lines.

The transposon-carrying MCG line is constructed by injecting the 3.2 kb *Xho*I fragment from the pMiCMVGFP plasmid into FVB X FVB fertilized oocytes. Transgenic founder animals are identified by Southern blotting of DNA from tail biopsies, using GFP DNA as a probe.

### Cell culture, transfection

13.5 day pregnant females (from crosses between MCG heterozygous transgenic male and wt females) are sacrificed, embryos are isolated and part of the material is used for genotyping. The remaining embryonic tissue is minced using a pair of scissors and immersed in a thin layer of F10/DMEM culture medium supplemented with 10 % FCS and antibiotics. Two spontaneously immortalized mouse embryonic fibroblasts lines (MEFs) with MCG/+ genotype are obtained by subculturing of primary MEFs. They are stably transfected with 20µg of plasmid pJGD/ILMi linearised with *Sca*I, using Lipofectin (GibcoBRL). Transfectants are selected on puromycin at a concentration of 1 µg/ml.

### Northern blot hybridisation

15 µg of total RNA isolated (Chomozynski & Sacchi (1987). Analytical Biochem. 162, 156-159) from kidney, thymus and spleen is subjected to electrophoresis in a 1.2% agarose gel containing 15% formaldehyde. Northern blot analysis is performed as described previously (Sambrook et al. (1989) Molecular Cloning. A Laboratory Manual. Cold Spring Harbor Laboratory Press, Cold Spring Harbor, NY)

### PCR analysis

Genomic DNA from different tissues is isolated with the DNeasy Tissue-Kit (QIAGEN) according to the manufacturers instructions. PCR reactions are performed using primers 11DML:
(5'AAGTGTAAGTGCTTGAAATGC-3')
and GOUM67:
(5'-GCATCAAATTGAGTTTTGCTC-3').

PCR conditions are as follows: 10 mM Tris-HCI (pH 8.8), 50 mM KCI, 1.5 mM MgCl₂, 0.001% gelatin; 1.2 units Taq 2000^{™} DNA Polymerase (STRATAGENE), 200 g template DNA and 10 pmol of each primer per. 25 µl reaction. 43 or 60 cycles or 30'' at 94°C, 30" at 59°C and 30" at 72°C were performed. PCR products are cloned into the PCRII TA cloning vector (Invitrogen) and are sequenced using the T7 primer.

### DNA fluorescent in situ hybridisation (FISH) analysis

Cells from minced thymus or spleen are cultured for 48h in RPMI medium (GIBCO BRL) supplemented with 9% FCS (GIBCO BRL), 13.6% Hybridoma medium (GIBCO BRL), 3.4 µg/ml Lithium chloride (MERCK), 7.2 µg/ml Concanavaline-A (SIGMA), 22.7 i.u./ml Heparine (LEO), 50 µM Mercaptoethanol, 25.4 µg/ml L.P.S. (SIGMA), 10 ng/ml interleukin 6 (PEPROTECH EC LTD). Chromosome preparations and FISH are carried out as described previously (Mulder et al. (1995) Hum Genet 96, 133-141. The 737 bp *Sac*I/*Not*I GFP fragment from the pMiCMVGFP construct is used as a probe. The probe is labelled with Biotin (Boehringer Manheim) and immunochemically detected with FITC. A telomeric probe for chromosome 14 (Shi, Y. et al. (1997) Genomics 45, 42-47) is labelled with dioxygenin (Boehringer Manheim) and immunochemically detected with Texas Red.

### Example 1: Activation of Minos in vivo in a tissue-specific manner

Two transgenic mouse lines are generated to determine whether *Minos* can transpose in mouse tissues: One containing a *Minos* transposon and another containing the *Minos* transposase gene expressed in a tissue-specific manner. The transposon-carrying line (line MCG) contains a tandem array of a fragment containing a *Minos* transposon (MiCMVGFP, figure 1) containing the GFP gene under the control of the cytomegalovirus promoter. The transposon is engineered such that almost all sequence internal to the inverted repeats is replaced by the CMV/GFP cassette. Not containing the transposase-encoding gene, this transposon is non-autonomous, and can only be mobilized when a source of transposase is present. The transposase-expressing line (line TM2) contains a tandem array of a construct comprising the *Minos* transposase cDNA under the control of the human CD2 locus, consisting of the CD2 promoter and LCR elements (pCD2/ILMi, figure 1). In transgenic mice, the human CD2 locus is transcribed at high levels in virtually all thymocytes as well as peripheral T cells (Zhumabekov, T. et al. (1995) J Immunol Methods 185, 113-140.

Heterozygous TM2/+ mice are tested for tissue-specific production of *Minos* transposase RNA by Northern blot analysis. *Minos* transposase mRNA is detected in thymus and spleen, the two organs with large numbers of T cells, but is not detected in other organs such as kidney (figure 2).

A PCR assay for transposon excision is used to detect active transposition by *Minos* transposase in mouse tissues, using primers that hybridise to the non-mobile *Drosophila hydei* sequences which flank the *Minos* transposon in the constructs shown in figure 1 (Klinakisv et al. (2000) Ins. Mol. Biol. 9, 269-275). In *Drosophila* cells, transposase-mediated excision of *Minos* is followed by repair of the chromatid which usually leaves a characteristic 6-base pair footprint (Arca et al. (1997) Genetics 145, 267-279). With the specific pair of primers used in the PCR assay this creates a diagnostic 167 bp PCR fragment (Catteruccia F. et al. (2000) Proc. Natl. Acad. Sci. U S A 97,2157-2162). As shown in figure 3, the diagnostic band is present in tissues of double transgenic (MCG/+ TM2/+) mice expressing the transposase, but not of MCG/+ mice, not expressing transposase. The identity of the fragment is confirmed by Southern blot analysis using a labelled DNA probe specific for the amplified sequence (data not shown). Excision is detectable mainly in thymus and spleen of the double transgenics; lower levels of excision are detectable in liver (figure 3). Very low levels of excision can also be detected in kidney, brain, and skeletal muscle, after 15 additional cycles of amplification (data not shown). Low levels of expression of the human CD2 locus in liver and lung of transgenic mice has been documented previously (Lang, G. et al. (1988) EMBO J . 6, 1675-1682). We therefore attribute the excision detected in tissues other than thymus and spleen to the presence of small numbers of T cells or to the expression of transposase in non T cells of these tissues due to position effects.

### Example 2: Detection of transposition in cultured embryonic fibroblasts.

The PCR excision assay is used to detect *Minos* excision in cultured embryonic fibroblasts carrying the MCG transgene. Cells are transfected with a plasmid carrying the *Minos* transposase cDNA under CMV control (pJGD/ILMi, figure 1) and analysed by the PCR excision assay. Excision products are detectable in transfected but not in non-transfected cells (data not shown). This result suggests that the transposon transgene is accessible to the *Minos* transposase in tissues other than T cells.

### Example 3: Detection of excision events.

To determine the nature of the excision events, PCR products from thymus and spleen of MCG/+ TM2/+ mice and from pJGD/ILMi transfected embryonic fibroblasts are cloned and sequenced. The sequence left behind after *Minos* excision in *Drosophila* consists of the TA dinucleotide duplication that is created upon *Minos* insertion, flanking the terminal 4 nucleotides of the transposon (i.e. either a AcgagT or a ActcgT insertion in the TA target site). In the mouse excisions analysed, the size and sequence of the footprints varies considerably (figure 4). Only 2 of the 32 footprints have the typical 6 bp sequence; the others contain extra nucleotides, in addition to complete or partial versions of the typical footprint. Four events have 1-2 nucleotides of the flanking *D. hydei* chromosomal sequence deleted. The differences in footprint structures observed between *Drosophila* and mouse may reflect the involvement of host factors in *Minors* excision and/or chromatid repair following excision.

### Example 4: Detection of transposition in transgenic mice using FISH

Detection of transposase-dependent excision in thymus and spleen suggests that transposition may also take place in these tissues. The detection of transposition events is not straightforward, because every transposition event is unique, and as a result the tissue in which transposition has occurred will be a mosaic of cells with unique transpositions. Indeed, Southern analysis did not show transposition events in the thymus of double transgenics, indicating that, if such mosaics exists, they consist of small numbers of clonally related cells.

Therefore, FISH in metaphase nuclei from the thymus and spleen to detect individual transposition events. A GFP fragment is used as a probe to detect relocalisation of transposons into new chromosomal positions. The initial position of the array of transposons is at the tip of chromosome 14, at a position indistinguishable from the telomere, as shown by co-localization, in metaphase and interphase chromosome, with a probe specific for telomeric sequences of chromosome 14 (figure 5, A-B). A total of 3,114 metaphases from 5 MCG/+ TM2/+ mice are analysed; 1,688 are from spleen and 1,426 from thymus. Nineteen of these metaphases (11 from spleen and 8 from thymus) show transposition. In addition to the signal at the tip of chromosome 14, pairs of dots are present in these metaphases on chromosomes other than 14, or on a new position on chromosome 14. Representative metaphases are shown in figure 5 (C-F). Morphological analysis of the chromosomes carrying new insertions show that all events except one are independent from each other, *i*.*e*. they represent different transpositions. Analysis of the positive metaphase with a probe specific for the telomere of chromosome 14 indicates that transpositions do not involve translocation of telomeric material (data not shown). As controls, 2,440 metaphases from thymus and spleens of five MCG/+ mice are screened; no transpositions are detectable in those samples.

This is the first demonstration that a transposase expressed from a transgene can mobilize a transposon to jump into new chromosomal sites in mammalian tissues.

## Claims

1. A method for generating a transgenic non-human mammalian organism, comprising the steps of:
(a) providing a first transgenic organism, which organism comprises, within at least a portion of its tissues or cells, as a result of transgenesis, one or more copies of a transposon;
(b) providing a second organism, which organism comprise, in the genome of at least a portion of its tissues or cells, as a result of transgenesis, a transposase or one or more copies of a gene encoding a transposase wherein the transposase is expressed under the control of control sequences which permit regulation of the expression of the transposase; and
(c) crossing the organisms so as to obtain transgenic progeny which comprise, in at least a portion of their tissues or cells, both the transposon and the transposase.

2. A method according to claim 1 wherein the regulation of expression of the transposase induces excision of a transposon.

3. A method according to claim 1 or claim 2 wherein the transposase is effective to activate transposition of a transposon to include excision of a transposon from a first integration site and/or integration of a transposon at a second integration site.

4. A method according to any preceding claim wherein excision of the transposon from a first integration site and/or integration of a transposon at the second integration site is used to genetically manipulate an organism.

5. A method according to any preceding claim, wherein the transposon has been modified to include a heterologous nucleic acid sequence, flanked by inverted terminal repeats homologous to the transposon.

6. A method according to claim 5, wherein the heterologous nucleic acid sequence is an enhancer or other transcriptional activation element.

7. A method according to any preceding claim, wherein the transposon has inserted therein a highly-transcribed gene such that, upon integration, the transposon lies in open chromatin structure.

8. A method according to any preceding claim, wherein the transposon is inserted into a highly expressed gene.

9. A method according to claim 8 wherein the transposon is inserted by recombination in ES cells.

10. A method according to any preceding claim, wherein the transposon is selected from the group consisting of Minos, mariner, Hermes and piggyBac.

11. A method according to claim 10, wherein the transposon is Minos.

12. A method according to any preceding claim, wherein the transposon comprises a nucleic acid sequence encoding a selectable marker.

13. A method according to claim 12, wherein the selectable marker is a fluorescent or luminescent polypeptide.

14. A method according to any preceding claim, wherein the transposase has been modified to optimise codon usage.

15. A method according to any preceding claim, wherein the control sequences are selected from inducible expression systems including tetracycline, oestrogen and ecdysone inducible systems.

16. A method according to claim 15, wherein the promoter is regulated in response to tissue-specific signals, developmental signals or exogenous signals.

17. A method according to any preceding claim, wherein the control sequences comprise a locus control region.

18. A method according to any preceding claim wherein the genetic manipulation of an organism includes the reversion of gene disruptions.

19. A method of detecting and characterising a genetic mutation in a transgenic organism, comprising the steps of:
(a) generating a transgenic non-human mammalian organism by a procedure according to any preceding claim;
(b) characterising the phenotype of the transgenic organism;
(c) detecting the position of one or more transposon insertion events in the genome of the organism; and
(d) correlating the position of the insertion events with the observed phenotype, the position of the insertion events being indicative of the location of one or more gene loci connected with the observed phenotype.

20. A method for isolating a gene which is correlated with a phenotypic characteristic in a transgenic animal, comprising the steps of:
(a) generating a transgenic non-human mammalian organism by a procedure according to any one of claims 1 to 18;
(b) characterising the phenotype of the transgenic organism;
(c) detecting the position of one or more transposon insertion events in the genome of the organism; and
(d) cloning the genetic loci comprising the insertions.

21. A method for isolating an enhancer in a transgenic non-human mammalian animal, comprising the steps of:
(a) generating a transgenic organism by a procedure according to any one of claims 1 to 18, wherein the transposon comprises a reporter gene under the control of a minimal promoter such that it is expressed at a basal level;
(b) assessing the level of expression of the reporter gene in one or more tissues of the transgenic organism;
(c) identifying and cloning genetic loci in which the modulation of the reporter gene is increased or decreased compared to the basal expression level; and
(d) characterising the cloned genetic loci.

22. A method for isolating an enhancer in a transgenic non-human mammalian animal, comprising the steps of:
(a) generating a transgenic organism by a procedure according to any one of claims 1 to 18, wherein the transposon comprises a reporter gene which lacks translation initiation sequences but includes splice acceptor sequences;
(b) identifying tissues of the organism in which the reporter gene is expressed;
(c) cloning the genetic loci comprising the expressed reporter gene.

23. A method for isolating an exon in a transgenic non-human mammalian animal, comprising the steps of:
(a) generating a transgenic organism by a procedure according to any one of claims 1 to 18, wherein the transposon comprises a reporter gene under the control of a minimal promoter such that it is expressed at a basal level;
(b) assessing the level of expression of the reporter gene in one or more tissues of the transgenic organism;
(c) identifying and cloning genetic loci in which the modulation of the reporter gene is increased or decrease compared to the basal expression level; and
(d) characterising the cloned genetic loci.

24. A method for isolating an exon in a transgenic non-human mammalian animal, comprising the steps of:
(a) generating a transgenic organism by a procedure according to any one of claims 1 to 18, wherein the transposon comprises a reporter gene which lacks translation initiation sequences but includes splice acceptor sequences;
(b) identifying tissues of the organism in which the reporter gene is expressed;
(c) cloning the genetic loci comprising the expressed reporter gene.

25. A method for modulating the expression of a gene in a non-human mammalian animal, comprising the steps of:
(a) generating a library of transgenic organisms by a method according to any of claims 1 to 18;
(b) selecting from said library one or more transgenic organisms in which the expression of a gene of interest is modulated as a result of one or more transposon insertion events.

## Patentansprüche

1. Verfahren zum Erzeugen eines transgenen, nicht-menschlichen Säugerorganismus, welches die folgenden Stufen umfaßt:
(a) Bereitstellen eines ersten transgenen Organismus, welcher infolge von Transgenese innerhalb wenigstens eines Teils seiner Gewebe oder Zellen eine oder mehrere Kopien eines Transposons umfaßt,
(b) Bereitstellen eines zweiten Organismus, welcher infolge von Transgenese im Genom wenigstens eines Teils seiner Gewebe oder Zellen eine Transposase oder eine oder mehrere Kopien eines Gens umfaßt, welches eine Transposase codiert, wobei die Transposase unter der Kontrolle von Kontrollsequenzen exprimiert wird, die die Regulation der Expression der Transposase gestatten, und
(c) Kreuzen der Organismen unter Erhalt transgener Nachkommen, die in wenigstens einem Teil ihrer Gewebe oder Zellen sowohl das Transposon als auch die Transposase umfassen.

2. Verfahren nach Anspruch 1, wobei die Regulation der Expression der Transposase die Exzision eines Transposons induziert.

3. Verfahren nach Anspruch 1 oder Anspruch 2, wobei die Transposase wirksam ist, um die Versetzung eines Transposons so zu aktivieren, daß sie die Exzision eines Transposons von einer ersten Integrationsstelle und/oder die Integration eines Transposons an einer zweiten Integrationsstelle beinhaltet.

4. Verfahren nach einem der vorangegangenen Ansprüche, wobei die Exzision des Transposons von einer ersten Integrationsstelle und/oder die Integration eines Transposons an der zweiten Integrationsstelle verwendet wird, um einen Organismus genetisch zu manipulieren.

5. Verfahren nach einem der vorangegangenen Ansprüche, wobei das Transposon so modifiziert wurde, daß es eine heterologe Nukleinsäuresequenz umfaßt, die durch invertierte terminale Wiederholungen flankiert ist, welche zu dem Transposon homolog sind.

6. Verfahren nach Anspruch 5, wobei die heterologe Nukleinsäuresequenz ein Enhancer oder ein anderes Transkriptionsaktivierungselement ist.

7. Verfahren nach einem der vorangegangenen Ansprüche, wobei in das Transposon ein hochgradig transkribiertes Gen eingebracht ist, so daß bei Integration das Transposon in einer offenen Chromatinstruktur liegt.

8. Verfahren nach einem der vorangegangenen Ansprüche, wobei das Transposon in ein hochgradig exprimiertes Gen eingebracht wird.

9. Verfahren nach Anspruch 8, wobei das Transposon durch Rekombination in ES-Zellen eingebracht wird.

10. Verfahren nach einem der vorangegangenen Ansprüche, wobei das Transposon aus der Gruppe ausgewählt ist, bestehend aus Minos, mariner, Hermes und piggyBac.

11. Verfahren nach Anspruch 10, wobei das Transposon Minos ist.

12. Verfahren nach einem der vorangegangenen Ansprüche, wobei das Transposon eine Nukleinsäuresequenz umfaßt, die einen selektierbaren Marker codiert.

13. Verfahren nach Anspruch 12, wobei der selektierbare Marker ein fluoreszierendes oder lumineszierendes Polypeptid ist.

14. Verfahren nach einem der vorangegangenen Ansprüche, wobei die Transposase so modifiziert wurde, daß die Codonenverwendung optimiert wird.

15. Verfahren nach einem der vorangegangenen Ansprüche, wobei die Kontrollsequenzen unter induzierbaren Expressionssystemen, einschließlich Tetrazyklin-, Östrogen- und Ecdyson-induzierbaren Systemen, ausgewählt sind.

16. Verfahren nach Anspruch 15, wobei der Promotor in Reaktion auf gewebespezifische Signale, Entwicklungssignale oder exogene Signale reguliert wird.

17. Verfahren nach einem der vorangegangenen Ansprüche, wobei die Kontrollsequenzen eine Locus-Kontrollregion umfassen.

18. Verfahren nach einem der vorangegangenen Ansprüche, wobei die genetische Manipulation eines Organismus die Umkehrung von Genunterbrechungen umfaßt.

19. Verfahren zum Detektieren und Charakterisieren einer genetischen Mutation in einem transgenen Organismus, welches die folgenden Stufen umfaßt:
(a) Erzeugen eines transgenen, nicht-menschlichen Säugerorganismus durch ein Verfahren nach einem der vorangegangenen Ansprüche,
(b) Charakterisieren des Phänotyps des transgenen Organismus,
(c) Detektieren der Position eines oder mehrerer Transposon-Insertionsereignisse im Genom des Organismus und
(d) Korrelieren der Position der Insertionsereignisse mit dem beobachteten Phänotyps, wobei die Position der Insertionsereignisse die Lage eines oder mehrerer Genloci anzeigt, die mit dem beobachteten Phänotyp in Zusammenhang stehen.

20. Verfahren zum Isolieren eines Gens, welches mit einem phänotypischen Merkmal in einem transgenen Tier korreliert ist, wobei das Verfahren die folgenden Stufen umfaßt:
(a) Erzeugen eines transgenen, nicht-menschlichen Säugerorganismus durch ein Verfahren nach einem der Ansprüche 1 bis 18,
(b) Charakterisieren des Phänotyps des transgenen Organismus,
(c) Detektieren der Position eines oder mehrerer Transposon-Insertionsereignisse in dem Genom des Organismus und
(d) Klonieren der genetischen Loci, welche die Insertionen umfassen.

21. Verfahren zum Isolieren eines Enhancers in einem transgenen, nicht-menschlichen Säugetier, wobei das Verfahren die folgenden Stufen umfaßt:
(a) Erzeugen eines transgenen Organismus durch ein Verfahren nach einem der Ansprüche 1 bis 18, wobei das Transposon ein Reportergen umfaßt, welches der Kontrolle eines minimalen Promotors untersteht, so daß es auf einem Grundniveau exprimiert wird,
(b) Ermitteln des Grades der Expression des Reportergens in einem oder mehreren Geweben des transgenen Organismus,
(c) Identifizieren und Klonieren genetischer Loci, worin die Modulation des Reportergens im Vergleich zum Grundniveau der Expression gesteigert oder verringert ist, und
(d) Charakterisieren der klonierten genetischen Loci.

22. Verfahren zum Isolieren eines Enhancers in einem transgenen, nicht-menschlichen Säugetier, wobei das Verfahren die folgenden Stufen umfaßt:
(a) Erzeugen eines transgenen Organismus durch ein Verfahren nach einem der Ansprüche 1 bis 18, wobei das Transposon ein Reportergen umfaßt, dem Translationsinitiationssequenzen fehlen, welches jedoch Spleiß-Akzeptorsequenzen beinhaltet,
(b) Identifizieren von Geweben des Organismus, in welchen das Reportergen exprimiert wird,
(c) Klonieren der genetischen Loci, die das exprimierte Reportergen umfassen.

23. Verfahren zum Isolieren eines Exons in einem transgenen, nicht-menschlichen Säugetier, wobei das Verfahren die folgenden Stufen umfaßt:
(a) Erzeugen eines transgenen Organismus durch ein Verfahren nach einem der Ansprüche 1 bis 18, wobei das Transposon ein Reportergen umfaßt, welches der Kontrolle eines minimalen Promotors untersteht, so daß es auf einem Grundniveau exprimiert wird,
(b) Ermitteln des Grades der Expression des Reportergens in einem oder mehreren Geweben des transgenen Organismus,
(c) Identifizieren und Klonieren genetischer Loci, in welchen die Modulation des Reportergens im Vergleich zum Grundniveau der Expression gesteigert oder verringert ist, und
(d) Charakterisieren der klonierten genetischen Loci.

24. Verfahren zum Isolieren eines Exons in einem transgenen, nicht-menschlichen Säugetier, wobei das Verfahren die folgenden Stufen umfaßt:
(a) Erzeugen eines transgenen Organismus durch ein Verfahren nach einem der Ansprüche 1 bis 18, wobei das Transposon ein Reportergen umfaßt, dem Translationsinitiationssequenzen fehlen, welches jedoch Spleiß-Akzeptorsequenzen beinhaltet,
(b) Identifizieren von Geweben des Organismus, in welchen das Reportergen exprimiert wird,
(c) Klonieren der genetischen Loci, die das exprimierte Reportergen umfassen.

25. Verfahren zum Modulieren der Expression eines Gens in einem nicht-menschlichen Säugetier, wobei das Verfahren die folgenden Stufen umfaßt:
(a) Erzeugen einer Bibliothek von transgenen Organismen durch ein Verfahren nach einem der Ansprüche 1 bis 18,
(b) Auswählen eines oder mehrerer transgener Organismen aus der Bibliothek, bei denen die Expression eines interessierenden Gens infolge eines oder mehrerer Transposon-Insertionsereignisse moduliert ist.

## Revendications

1. Procédé pour générer un organisme mammifère transgénique non humain, comprenant les étapes consistant à :
(a) fournir un premier organisme transgénique, lequel organisme comprend, dans au moins une partie de ses tissus ou cellules, suite à une transgenèse, un ou plusieurs exemplaires d'un transposon ;
(b) fournir un second organisme, lequel organisme comprend, dans le génome d'au moins une partie de ses tissus ou cellules, suite à une transgenèse, une transposase ou un ou plusieurs exemplaires d'un gène codant une transposase, la transposase étant exprimée sous le contrôle de séquences de contrôle qui permettent la régulation de l'expression de la transposase ; et
(c) croiser les organismes de manière à obtenir une descendance transgénique qui comprend, dans au moins une partie de ses tissus ou cellules, le transposon et la transposase.

2. Procédé selon la revendication 1 dans lequel la régulation de l'expression de la transposase entraîne l'excision d'un transposon.

3. Procédé selon la revendication 1 ou la revendication 2 dans lequel la transposase est efficace pour activer la transposition d'un transposon pour inclure l'excision d'un transposon à partir d'un premier site d'intégration et/ou l'intégration d'un transposon au niveau d'un second site d'intégration.

4. Procédé selon l'une quelconque des revendications précédentes dans lequel l'excision du transposon à partir d'un premier site d'intégration et/ou l'intégration d'un transposon au niveau du second site d'intégration est utilisée pour manipuler génétiquement un organisme.

5. Procédé selon l'une quelconque des revendications précédentes, dans lequel le transposon a été modifié pour inclure une séquence d'acide nucléique hétérologue, flanquée par des répétitions terminales inversées homologues au transposon.

6. Procédé selon la revendication 5, dans lequel la séquence d'acide nucléique hétérologue est un amplificateur ou un autre élément d'activation de la transcription.

7. Procédé selon l'une quelconque des revendications précédentes, dans lequel un gène hautement transcrit est inséré dans le transposon de sorte que, lors de l'intégration, le transposon se trouve dans une structure de chromatine ouverte.

8. Procédé selon l'une quelconque des revendications précédentes, dans lequel le transposon est inséré dans un gène hautement exprimé.

9. Procédé selon la revendication 8 dans lequel le transposon est inséré par recombinaison dans des cellules ES.

10. Procédé selon l'une quelconque des revendications précédentes, dans lequel le transposon est choisi dans le groupe consistant en Minos, mariner, Hermes et piggyBac.

11. Procédé selon la revendication 10, dans lequel le transposon est Minos.

12. Procédé selon l'une quelconque des revendications précédentes, dans lequel le transposon comprend une séquence d'acide nucléique codant un marqueur sélectionnable.

13. Procédé selon la revendication 12, dans lequel le marqueur sélectionnable est un polypeptide fluorescent ou luminescent.

14. Procédé selon l'une quelconque des revendications précédentes, dans lequel la transposase a été modifiée pour optimiser l'utilisation du codon.

15. Procédé selon l'une quelconque des revendications précédentes, dans lequel les séquences de contrôle sont choisies parmi des systèmes d'expression inductibles incluant les systèmes inductibles par la tétracycline, l'oestrogène et l'ecdysone.

16. Procédé selon la revendication 15, dans lequel le promoteur est régulé en réponse à des signaux spécifiques de tissu, des signaux développementaux ou des signaux exogènes.

17. Procédé selon l'une quelconque des revendications précédentes, dans lequel les séquences de contrôle comprennent une région de contrôle de locus.

18. Procédé selon l'une quelconque des revendications précédentes dans lequel la manipulation génétique d'un organisme inclut l'inversion de disruptions géniques.

19. Procédé pour détecter et caractériser une mutation génétique dans un organisme transgénique, comprenant les étapes consistant à :
(a) générer un organisme mammifère transgénique non humain par une procédure selon l'une quelconque des revendications précédentes ;
(b) caractériser le phénotype de l'organisme transgénique ;
(c) détecter la position d'un ou plusieurs événements d'insertion de transposon dans le génome de l'organisme ; et
(d) corréler la position des événements d'insertion au phénotype observé, la position des événements d'insertion indiquant l'emplacement d'un ou plusieurs locus génétiques liés au phénotype observé.

20. Procédé pour isoler un gène qui est corrélé à une caractéristique phénotypique chez un animal transgénique, comprenant les étapes consistant à :
(a) générer un organisme mammifère transgénique non humain par une procédure selon l'une quelconque des revendications 1 à 18 ;
(b) caractériser le phénotype de l'organisme transgénique ;
(c) détecter la position d'un ou plusieurs événements d'insertion de transposon dans le génome de l'organisme ; et
(d) cloner les locus génétiques comprenant les insertions.

21. Procédé pour isoler un amplificateur dans un animal mammifère transgénique non humain, comprenant les étapes consistant à :
(a) générer un organisme transgénique par une procédure selon l'une quelconque des revendications 1 à 18, le transposon comprenant un gène rapporteur sous le contrôle d'un promoteur minimal de manière à être exprimé à un niveau de base ;
(b) évaluer le niveau d'expression du gène rapporteur dans un ou plusieurs tissus de l'organisme transgénique ;
(c) identifier et cloner les locus génétiques dans lesquels la modulation du gène rapporteur est accrue ou diminuée comparativement au niveau d'expression de base ; et
(d) caractériser les locus génétiques clonés.

22. Procédé pour isoler un amplificateur dans un animal mammifère transgénique non humain, comprenant les étapes consistant à :
(a) générer un organisme transgénique par une procédure selon l'une quelconque des revendications 1 à 18, le transposon comprenant un gène rapporteur qui ne contient pas de séquences de lancement de la traduction mais inclut des séquences acceptrices d'épissage ;
(b) identifier des tissus de l'organisme dans lesquels le gène rapporteur est exprimé ;
(c) cloner les locus génétiques comprenant le gène rapporteur exprimé.

23. Procédé pour isoler un exon dans un animal mammifère non humain transgénique, comprenant les étapes consistant à :
(a) générer un organisme transgénique par une procédure selon l'une quelconque des revendications 1 à 18, dans lequel le transposon comprend un gène rapporteur sous le contrôle d'un promoteur minimal de manière à ce qu'il soit exprimé à un niveau de base ;
(b) évaluer le niveau d'expression du gène rapporteur dans un ou plusieurs tissus de l'organisme transgénique ;
(c) identifier et cloner les locus génétiques dans lesquels la modulation du gène rapporteur est accrue ou diminuée comparativement au niveau d'expression de base ; et
(d) caractériser les locus génétiques clonés.

24. Procédé pour isoler un exon dans un animal mammifère transgénique non humain, comprenant les étapes consistant à :
(a) générer un organisme transgénique par une procédure selon l'une quelconque des revendications 1 à 18, le transposon comprenant un gène rapporteur qui ne contient pas de séquences de lancement de la traduction mais inclut des séquences acceptrices d'épissage ;
(b) identifier les tissus de l'organisme dans lesquels le gène rapporteur est exprimé ;
(c) cloner les locus génétiques comprenant le gène rapporteur exprimé.

25. Procédé pour moduler l'expression d'un gène dans un animal mammifère non humain, comprenant les étapes consistant à :
(a) générer une banque d'organismes transgéniques par un procédé selon l'une quelconque des revendications 1 à 18 ;
(b) sélectionner dans ladite banque un ou plusieurs organismes transgéniques dans lesquels l'expression d'un gène d'intérêt est modulée suite à un ou plusieurs événements d'insertion de transposon.
